# EUROPEAN PATENT SPECIFICATION

(11) **EP 1 924 597 B1**
(45) Date of publication and mention of the grant of the patent: **02.05.2012**
(21) Application number: 06792095.9
(22) Date of filing: 15.09.2006
(51) Int. Cl.: C07K 14/02, A61K 39/39, A61P 31/04

(54) **VACCINES COMPRISING TRUNCATED HBC CORE PROTEIN PLUS SAPONIN-BASED ADJUVANTS**
TRUNKIERTES HBC CORE-PROTEIN PLUS EIN SAPONIN-BASIERENDES ADJUVANS ENTHALTENDE VAKZINE
VACCINS COMPRENANT LA PROTÉINE DU NOYAU DU HBV TRONQUÉE AINSI QU'UN ADJUVANT À BASE DE SAPONINE

(30) Priority: 16.09.2005 EP 05020208
(43) Date of publication of application: 28.05.2008
(73) Proprietor: Rhein Biotech Gesellschaft für neue biotechnologische Prozesse und Produkte mbH, 40595 Düsseldorf (DE)
(72) Inventor: MELBER, Karl, 40591 Düsseldorf (DE); BUCHMANN, Pascale, 51061 Köln (DE); JANOWICZ, Zbigniew, 40699 Erkrath (DE)
(74) Representative: Herzog, Martin
(86) International application number: PCT/EP2006/009014
(87) International publication number: WO 2007/031334

(56) References cited:
- WO-A-2004/004762
- WO-A-2005/002620
- JP-A- 8 059 695
- CHEN XINCHUN ET AL: "Recombinant hepatitis B core antigen carrying preS1 epitopes induce immune response against chronic HBV infection." VACCINE, vol. 22, no. 3-4, 2 January 2004 (2004-01-02), pages 439-446, XP004479365 ISSN: 0264-410X
- PEARSE MARTIN J ET AL: "ISCOMATRIX(R) adjuvant for antigen delivery" ADVANCED DRUG DELIVERY REVIEWS, vol. 57, no. 3, 10 January 2005 (2005-01-10), pages 465-474, XP004653216 ISSN: 0169-409X
- AGUILAR J C ET AL: "Development of a nasal vaccine for chronic hepatitis B infection that uses the ability of hepatitis B core antigen to stimulate a strong Th1 response against hepatitis B surface antigen" IMMUNOLOGY AND CELL BIOLOGY, vol. 82, no. 5, October 2004 (2004-10), pages 539-546, XP002354413 ISSN: 0818-9641
- HOWARD C R ET AL: "PREPARATION AND PROPERTIES OF IMMUNE-STIMULATING COMPLEXES CONTAINING HEPATITIS B VIRUS SURFACE ANTIGEN" JOURNAL OF GENERAL VIROLOGY, vol. 68, no. 9, 1987, pages 2281-2289, XP009057159 ISSN: 0022-1317
- GATHURU ET AL: "Identification of DHBcAg as a potent carrier protein comparable to KLH for augmenting MUC1 antigenicity" VACCINE, BUTTERWORTH SCIENTIFIC. GUILDFORD, GB, vol. 23, no. 39, 15 September 2005 (2005-09-15), pages 4727-4733, XP005040630 ISSN: 0264-410X
- POLAKOS N K ET AL: "Characterization of hepatitis C virus core-specific immune responses primed in rhesus macaques by a nonclassical ISCOM vaccine." JOURNAL OF IMMUNOLOGY (BALTIMORE, MD. : 1950) 1 MAR 2001, vol. 166, no. 5, 1 March 2001 (2001-03-01), pages 3589-3598, XP002354377 ISSN: 0022-1767
- GUAN XIAO-JU ET AL: "Construction and characterization of an experimental ISCOMS-based hepatitis B polypeptide vaccine." WORLD JOURNAL OF GASTROENTEROLOGY : WJG. APR 2002, vol. 8, no. 2, April 2002 (2002-04), pages 294-297, XP002354578 ISSN: 1007-9327
- NEWMAN MARGARET ET AL: "Stability and morphology comparisons of self-assembled virus-like particles from wild-type and mutant human hepatitis B virus capsid proteins." JOURNAL OF VIROLOGY, vol. 77, no. 24, December 2003 (2003-12), pages 12950-12960, XP002354380 ISSN: 0022-538X
- TSAI S L: "Immunopathogenesis of viral hepatitis B and C." CHANGGENG YI XUE ZA ZHI / CHANGGENG JI NIAN YI YUAN = CHANG GUNG MEDICAL JOURNAL / CHANG GUNG MEMORIAL HOSPITAL. JUN 1999, vol. 22, no. 2, June 1999 (1999-06), pages 159-170, XP009057239
- RIEDL PETRA ET AL: "Priming Th1 immunity to viral core particles is facilitated by trace amounts of RNA bound to its arginine-rich domain" JOURNAL OF IMMUNOLOGY, vol. 168, no. 10, 15 May 2002 (2002-05-15), pages 4951-4959, XP002354381 ISSN: 0022-1767
- MOREIN BROR ET AL: "Current status and potential application of ISCOMs in veterinary medicine" ADVANCED DRUG DELIVERY REVIEWS, vol. 56, no. 10, 23 June 2004 (2004-06-23), pages 1367-1382, XP002354382 ISSN: 0169-409X
- SCHAEFER-S ET AL.: "Recombinant hepatitis B Vaccines - Disease Characterization and Vaccine Production" 28 January 2005 (2005-01-28), , IN: HANSENULA POLYMORPHA; ED: GERD GELLISSEN; PRINT ISBN-10: 3527303413; ONLINE ISBN-10: 3527602356; P 175-210 , XP002411804 the whole document
- SCHIRMBECK R ET AL: "DIFFERENT IMMUNOGENCITIY OF H-2 KB-RESTRICTED EPITOPES IN NATURAL VARIANTS OF THE HEPATITIS B SURFACE ANTIGEN" EUROPEAN JOURNAL OF IMMUNOLOGY, WEINHEIM, DE, vol. 33, no. 9, September 2003 (2003-09), pages 2429-2438, XP009040727 ISSN: 0014-2980
- SCHIRMBECK R ET AL: "BREAKING TOLERANCE IN HEPATITIS B SURFACE ANTIGEN (HBSAG) TRANSGENIC MICE BY VACCINATION WITH CROSS-REACTIVE, NATURAL HBSAG VARIANTS" EUROPEAN JOURNAL OF IMMUNOLOGY, WEINHEIM, DE, vol. 33, no. 12, December 2003 (2003-12), pages 3342-3352, XP009040725 ISSN: 0014-2980
- MANCINI M ET AL: "INDUCTION OF ANTI-HEPATITIS B SURFACE ANTIGEN (HBSAG) ANTIBODIES IN HBAAG PRODUCING TRANSGENIC MICE: A POSSIBLE WAY OF CIRCUMVENTING NONRESPONSE TO HBSAG" JOURNAL OF MEDICAL VIROLOGY, ALAN R. LISS, NEW YORK, NY, US, vol. 39, no. 1, 1993, pages 67-74, XP009040798 ISSN: 0146-6615
- DAVIS H L ET AL: "DNA-based immunization against hepatitis B surface antigen (HBsAg) in normal and HBsAg-transgenic mice" VACCINE, BUTTERWORTH SCIENTIFIC. GUILDFORD, GB, vol. 15, no. 8, June 1997 (1997-06), pages 849-852, XP004075668 ISSN: 0264-410X
- MAGNIUS L O ET AL: "SUBTYPES, GENOTYPES AND MOLECULAR EPIDEMIOLOGY OF THE HEPATITIS B VIRUS AS REFLECTED BY SEQUENCE VARIABILITY OF THE S-GENE.ATITIS B" INTERVIROLOGY, vol. 38, no. 1/2, 1995, pages 24-34, XP000197124 ISSN: 0300-5526
- MARC HECKMANN ET AL: "Letter: Botulinum Toxin Overrides Depression: Not Surprising, yet Sensational", Dermatologic Surgery, vol. 33, no. 6, 1 June 2007 (2007-06-01), pages 765-765, XP55003857, ISSN: 1076-0512, DOI: 10.1111/j.1524-4725.2007.33160.x

### SEQUENCE LISTING

<110> Rhein Biotech Gesellschaft für neue biotechnologische Prozesse und Produkte mbH
<120> A composition for therapy and/or prophylaxis of HBV-infections and HBV-mediated diseases
<130> RB82336PC
<150> EP2005020208.4
   <151> 2005-09-16
<160> 2
<170> PatentIn version 3.1
<210> 1
   <211> 8
   <212> PRT
   <213> synthetic polypeptide
<400> 1
   Met Gly Leu Lys Phe Arg Gln Leu
   1               5
<210> 2
   <211> 23
   <212> DNA
   <213> synthetic polynucleotide
<400> 2
   tccggagata tcgatcgtcc gga 23

## Description

The present invention relates to a composition, to a process for production of a composition, to the composition obtainable by this process, to a pharmaceutical formulation, to the use of the composition or of the pharmaceutical formulation for therapy and/or prophylaxis of hepatitis B virus (HBV)-infections and HBV-mediated diseases, to the use of the composition or of the pharmaceutical formulation for production of a pharmaceutical for therapy and/or for prophylaxis of HBV-infections and HBV-mediated diseases as well as to a process for therapy and/or for prophylaxis of HBV-infections and HBV-mediated diseases.

According to World Health Organization (WHO) estimates, more than 2 billion of the people living today are or have been infected with the hepatitis B virus (HBV). HBV thus belongs to the most significant human pathogenic agents with enormous effects on human health.

Infections with HBV occur via blood, in particular from the infected mother to the newborn during or shortly after birth, by means of contaminated blood products and through sexual intercourse. HBV represents a hepatotropic virus, which can cause both acute and chronic infections. While an infection at birth or in the first month of life leads, in 90 % of cases, to a chronic infection, this is only observed in about 10 % of cases with infection in adulthood. In about 2 % of infected adults, a fulminant hepatitis (acute liver failure) can occur, which is linked to a high death rate.

At first, a chronic infection is without clinical symptoms, but can develop after a latency period of 10 to 30 years into an acute liver disease (active, chronic hepatitis B) because of the persisting HBV and the pathological interaction of the immune system with the infected liver cells. The number of chronic HBV carriers who develop chronic hepatitis B is around 25 %. Chronic hepatitis B can finally lead to liver cirrhosis and/or primary liver cell carcinoma. Both diseases are linked with a high death rate. It is estimated that there are currently around 350 million chronically infected HBV-carriers worldwide. Every year, around a million people die from the consequences of a chronic HBV-infection.

Fortunately, HBV infections can be prevented by prophylactic hepatitis B vaccines. Such vaccines, based on purified hepatitis B surface antigen (HBsAg) from the plasma of chronic carriers were first available at the start of the 1980's. Later, these were replaced by HBsAg produced recombinantly. Since, however, vaccination is not currently routinely introduced in all countries, HBV will continue to represent a threat to human health. In this context, it is significant that chronic HBV carriers are considered as infectious and can transfer the virus.

Chronic hepatitis B is a very dynamic disease with a number of forms of appearance. In the early stages of the chronic infection, one talks of the immunotolerant phase, which is characterised by the presence of viral markers in the serum (HBV-DNA, HBsAg, HBeAg) as well as the lack of liver problems. Around 25 % of HBV carriers transfer into an immunoactive phase, in the course of which the first liver symptoms appear and a therapy becomes necessary. During this immunoactive phase, HBV-DNA in the serum generally decrease and serum-transaminases increase batchwise. While HBsAg continues to be present in the serum, there is a small percentage (around 1 %) of patients, which spontaneously undergoes a serum conversion from HBeAg positive to HBeAg negative. This serum conversion is a sign for the entry into the inactive carrier stage, which is characterised by a low virus activity. In few cases, a seroconversion from HBsAg positive to anti-HBsAg positive is additionally observed, which is considered a sign of a healed infection. The active chronic hepatitis B is characterised by necro-inflammation and fibrosis of the liver tissues, which leads in the course of time to cirrhosis and decompensated liver disease. As further complication, cirrhosis can lead to primary liver cell carcinoma, whereby some chronic HBV carriers can also develop liver cancer without signs of cirrhosis.

The treatment possibilities for active chronic hepatitis B are very limited. Currently, a treatment can take place with Interferon-α (for example Intron A^{®} or Roferon A^{®}) or antiviral substances (Lamivudine, Adefovir, Entecavir). Interferon-α is preferably used with HBeAg positive patients, whereby around 30 % react with a HBeAg-serum conversion, which correlates with an inactive carrier status. As a result, a few patients (around 3 %) also show an HBsAg seroconversion as sign of a full healing. The treatment with Interferon-α is, however, linked with severe side effects, which often necessitate an early stop to the therapy. In HBeAg negative patients, Interferon-α shows low response rates compared with HBeAg positive patients. As alternatives, antiviral substances, which belong to the class of nucleoside/nucleotide reverse transcriptase inhibitors, can be used here. These antiviral substances inhibit the replication of the virus and, as a result, prevent advance of the liver disease. The serum conversion rates for HBeAg positive patients are lower, compared with Interferon-α, and are not higher than the spontaneously occurring remission. The target of the antiviral therapy is thus to delay the disease by reduction of the viral activity for an unlimited time. The long-term use of the antiviral substances, is, however, jeopardised by the appearance of resistant virus mutants. In the case of the most frequently used substance, Lamivudine, a resistance rate of up to 68 % within four years has been observed. The most recently introduced, Adefovir, seems to cause resistance less frequently. The basic problem of virus resistance represents, however, an inherent threat for long-term treatment, even if the antiviral substances should be used in combination. There exists, therefore, an urgent need for additional therapeutic means of treating chronic hepatitis B. Particularly desirable are immunomodulatory agents with few side effects and with the perspective for a short therapy for the patients.

Such an alternative is the specific immunotherapy in the form of a therapeutic vaccine. The concept of a therapeutic vaccine is to stimulate specifically the immune system of a chronic hepatitis B patient in such a way that an adequate immune response is initiated against the virus, which finally leads to control of the virus and curbs the advance of the liver disease. One of the obvious effects which a therapeutic vaccine must show is the breaking through of the present immunotolerance in the presence of large amounts of viral antigens. The therapy should, in particular, generate virus-specific T-cell responses, in particular cytotoxic T-lymphocytes (CTL) against diverse antigens of HBV. Compared with the situation for acute, healing infection, in patients with chronic hepatitis B exactly these immune responses can only be shown to a very small extent, or not at all. Strong virus-specific CTL responses are generally viewed as representatives of the control of viral infections. The development of a therapeutic vaccine has, therefore, as primary target, the induction of strong antiviral CTL responses.

Recombinant subunit vaccines are generally not able to cause CTL responses. In animal models (mouse), significant CTL responses with DNA-vaccines, in particular DNA-vaccines based on a hepatitis B core antigen (HBcAg)-coding plasmid vector can be achieved, however, current vaccines show to date overall in humans only very weak immune response and do not stimulate any potent CTL response. Because of these difficulties, so-called "Prime-Boost-Vaccines" are currently being developed, whereby immunization takes place alternately with a DNA-vaccine (e.g. coding for HBsAg) and a recombinant virus vector (e.g. modified Vaccinia Ankara Stem), which, e.g. expresses the HBsAg (see McConkey S.J., Schneider J., Mendy M., Cavenaugh J.S., Bertoletti A., Whittle H., Hill A.V.S.: "Safety and Immunogenicity of a novel "prime boost" therapeutic immunization strategy against hepatitis B virus", Meeting abstract: "The molecular biology of hepatitis B viruses", Bergamo, Italy, September 7-10, 2003).

WO-A-01/98333 describes the use of an HBc-antigen, in which one or more of the four arginine repeat units at the C-terminus was removed, but the C-terminal cysteine residue was retained, *inter alia* for therapeutic treatment of HBV. The removed arginine repeat units can be replaced by sequences which represent epitopes from bacterial or viral pathogens, parasites, allergens or antigens associated with cancer, which are recognised by T-helper cells, B-cells or cytotoxic T-lymphocytes.

WO-A-2005/023297 describes a composition for treatment of HBV-infections and HBV-mediated diseases, which comprise at least two hepatitis B surface antigens (HBsAg's), fragments thereof and/or nucleic acids coding these, whereby the HBsAg's in the HBV genotype in the S-region and/or the pre-S1-region are different and whereby the composition comprises no HBcAg or nucleic acids coding this.

WO-A-2005/056051 discloses inter alia a recombinant polypeptide composition for treatment or prophylaxis of a hepatitis B infection, whereby the composition comprises a plurality of components, preferably at least three or all four selected from a) a core-polypeptide element, which comprises a sequence of the immunogenic region of the HBV-core-protein, b) a surface antigen-polypeptide element, which comprises a sequence of the immunogenic region of the HBV-surface antigen-protein (S, M or L), c) an X-polypeptide element, which comprises a sequence of the immunogenic region of the HBV-X-protein and d) a polymerase-polypeptide-element, which comprises a sequence of the immunogenic region of the HBV-polymerase-proteins, whereby at least one of the elements comprises at least 100 amino acids of the respective HBV proteins.

EP-A-1 136 077 discloses a vaccine composition for nasal application, comprising a HBsAg and optionally a HBcAg, which, *inter alia*, can also be used as therapeutic vaccines.

Chen Xinchun et al. (Vaccine, Vol. 22, Issues 3-4, pages 439-446, 2004) describe a recombinant hepatitis B core antigen carrying preS1 epitopes induce immune response against chronic HBV infection.

Pearse et al. (Advanced Drug Delivery Reviews, Vol. 57, Issue 3, pages 465-474, 2005) describe the use of ISCOMATRIX^{®} adjuvant for antigen delivery.

Aguilar et al. (Immunology and Cell Biology, Vol. 82, Issue 5, pages 539-546, 2004) deals with the development of a nasal vaccine for chronic hepatitis B infection that uses the ability of hepatitis B core antigen to stimulate a strong Th1 response against hepatitis B surface antigen.

JP 08 059695 A discloses a new polypeptide, having a specific amino acid sequence, capable of manifesting hepatitis B virus antigenicity and antigenicity close to that of a natural product.

Sons-HS et al. (Molecular Biology of the Cell, Vol. 7, Issue 2, pages 176-186, 1997) describes the effect of a novel saponin adjuvant derived from *Quillaja saponaria* on immune response to recombinant hepatitis B surface antigen.

Pumpens-P et al. (Intervirology, Vol. 44, Issues 2-3, pages 98-114, 2001) is directed to the use of HBV core particles as a Carrier for B Cell/T Cell Epitopes.

Boulter-NR et al. (Vaccine, Vol. 13, Issue 13, pages 1152-1160, 1995) discloses a fusion protein wherein *Theileria annulata* sporozoite antigen is fused to hepatitis B core antigen and the use of such a fusion protein in a vaccination trial.

The present invention has the object of overcoming the disadvantages known from the prior art for vaccines for treatment or prophylaxis of a hepatitis B infection in humans.

In particular, the present invention had the object of providing a composition suitable for treatment of a human, which, in comparison to the compositions known from the prior art, leads to strong immune responses, in particular strong CTL responses against the HBcAg, in patients with a chronic HBV infection. The composition should lead to breaking through of the immunological tolerance in as many patients as possible with a chronic HBV infection, irrespective of their HLA type.

The composition according to the invention should enable a breaking through of the immunological tolerance, irrespective of the HBV genotypes of the chronic patient and thus lead to generation of an immune response which keeps the virus under control.

The present invention further had the object of providing a process with which a composition with the above-described advantages can be obtained with as little preparative complexity as possible.

A contribution to the solution of the above-mentioned object is provided by a composition, comprising:
i) a HBcAg₁₋ₓ (HBcAg = HBV core-antigen), whereby x is a whole number from the range from 100 to 160, preferably from the range from 120 to 150, particularly preferably from the range from 140 to 149, yet more preferably or from the range from 144 to 146 and most preferably equal to 145, or a variant of this antigen,
ii) an adjuvant comprising a saponin wherein the adjuvant is a saponin complex, and
iii) a HBsAg or a variant of this antigen.

Completely surprisingly, however nonetheless advantageous, it was observed that, with the above-described composition, which is in principle suitable for a use in humans, strong, core-specific CTL responses can be obtained in the animal model, which were roughly comparable with those CTL responses which were obtained using a HBcAg-coding plasmid vector (DNA-immunisation) in the same animal model.

The scope of the present invention is defined by the claims and any information that does not fall within the claims is provided for information only The term "polypeptide" used in the following details relates to a polymer of amino acids and is not restricted to a particular minimum number of amino acids. It therefore comprises equally peptides, oligopeptides, dimers, trimers, oligomers, particles and the like. Furthermore, the term "polypeptides" comprises not only the pure polymers of amino acids, which are obtained directly after translation in the ribosomes, but also those polypeptides which are obtained post-translationally by glycosylation, acetylation, phosphorylation and the like.

By a "HBcAg₁₋ₓ" is understood according to the invention a polypeptide which comprises the amino acids 1 to x of the natural HBcAg, preferably which consists of the amino acids 1 to x of the natural HBcAg, whereby by the term "'HBeAg₁₋ₓ" should be understood all conceivable polypeptides with the amino acids 1 to x (whereby the first amino acid (= amino acid 1) is that at the N-terminal end of the polypeptide) of the HBcAg of the currently known 8 standard genotypes A, B, C, D, E, F, G and H. These 8 standard genotypes differ from each other in approximately 8 % of the nucleotide sequence (see Bartholomeusz, Rev. Med. Virol. 13 (2003), 1-14), whereby the variations relates predominantly to the HBsAg-gene. Preferably the HBV genotype A comprises the reference nucleic acid sequence Genbank X02763 or respectively the HBV genotype A_{afr} comprises the reference nucleic acid sequence according to Genbank AF297621. For the HBV genotype Bₐ the reference nucleic acid sequence is Genbank D00330, for the genotype Bⱼ, the reference nucleic acid sequence AB073858. For the HBV genotype C, the reference nucleic acid sequence is Genbank AY206389, for the genotype Cₐᵤₛ, the reference nucleic acid sequence according to Genbank AB048704. For the genotype D, the reference nucleic acid sequence is Genbank X02496, for the genotype E, Genbank X75657, for the genotype F, Genbank X69798, for the genotype G, Genbank AF160501 and for the genotype H, Genbank AY090454.

By the term "variant" of the HBcAg₁₋ₓ is understood a polypeptide in which at most 5 amino acids, particularly preferably at most 2 amino acids and most preferably at most 1 amino acid of the HBcAg₁₋ₓ has been deleted, inserted, substituted or appended at the C- and/or N-terminal ends, preferably substituted. The term "variants" also comprises fusion polypeptides, in particular fusions with HBsAg, which comprise HBcAg₁₋ₓ.

Under the term "adjuvant" is understood according to the invention a compound which enables the induction of an immune response, preferably the induction of a CTL response to the antigen.

The HBcAg₁₋ₓ's which are comprised in the composition according to the invention and which are truncated compared to the natural HBcAg's, of which at least a part of the C-terminal nucleic acid binding region, preferably the whole C-terminal nucleic acid binding region is lacking, can be obtained by recombination processes known to the skilled person.

One possibility is to amplify the core or pre-core reading frame from a virus isolate by means of polymerase chain reaction (PCR). It is also conceivable to prepare the corresponding DNA-sequence synthetically and then to amplify by means of PCR. The obtained PCR fragment is then cloned in a plasmid vector. By means of further PCR-cloning, the correspondingly shortened form can then be inserted into an expression vector, for example into an expression vector *E. coli*. Thereby, the insertion preferably occurs in such a way that the gene coding for HBcAg₁₋ₓ or for the fragment or the variant respectively is under the control of an active promoter. The expression vector can simultaneously comprise induction elements for increasing the expression rate. After the transformation of this expression vector in *E. coli*, individual clones are obtained, which express the HBcAg₁₋ₓ or the fragment or the variants respectively. In order to obtain the polypeptides which serve as antigens, an individual clone is injected into culture medium and cultivated overnight at 37°C in the shaking flask. The *E. coli* culture is then first harvested by centrifugation. The bacteria pellet is re-suspended in buffer solution and then disrupted either by ultrasound treatment or by high pressure homogenisation. The HBcAg or HBcAg-variants respectively situated in the homogenisate are concentrated by precipitation with ammonium sulphate and then purified of bacterial host components by gel filtration. Particle-forming HBcAg-variants can be further purified by a speed zone centrifugation. Particle-deficient HBcAg₁₋ₓ's are ideally engineered with an N-terminal His-Tag-affinity marker and purified by means of a Ni-NTA-affinity chromatography and subsequent gel filtration. The thus-produced HBcAg₁₋ₓ's are subjected to a sterile filtration and used as such for the formulation of the composition according to the invention.

In principal the HBcAg₁₋ₓ's or their variants comprised in the composition according to the invention can also the obtained synthetically.

Further details concerning techniques of recombinant or synthetic preparation of polypeptides can be found in
- Sambrook, "Molecular Cloning: A Laboratory Manual", second edition (1989);
- "DNA Cloning", Volumes I and II (D. N. Glover, editor., 1985);
- "Oligonucleotide Synthesis" (M. J. Gait, editor, 1986);
- "Nucleic Acid Hybridization" (B. D. Hames & S. J. Higgins, editors, 1984);
- "Transcription and Translation" (B. D. Hames & S. J. Higgins, editors, 1984);
- "Animal Cell Culture" (R. I. Freshney, editors, 1986);
- "Immobilised Cells and Enzymes" (IRL Press, 1986);
- B. Perbal, "A Practical Guide to Molecular Clonning" (1984);
- "The Methods in Enzymology series" (Academic Press, Inc.), in particular Volumes 154 and 155;
- "Gene Transfer Vectors for Mammalian Cells" (J. H. Miller and M. P. Calos, editors, 1987, Cold Spring Harbor Laboratory);
- Mayer und Walker, editors, (1987), "Immunochemical Methods in Cell and Molecular Biology" (Academic Press, London);
- Scopes, (1987), "Protein Purification: Principles and Practice", second edition (Springer-Verlag, N.Y.) and
- "Handbook of Experimental Immunology", Volumes I-IV (D. M. Weir and C. C. Blackwell, editors, 1986)

In principal, the HBcAg₁₋ₓ's comprised in the composition according to the invention can be particle forming or non particle forming, whereby particle forming HBcAg₁₋ₓ's are preferred. Particle forming HBcAg₁₋ₓ's can associate to form cap-sides with a diameter of approximately 27 nm. 180 Or 240 dimers associate to form macromolecular structures. According to a particularly preferred embodiment of the composition according to the invention, the HBcAg₁₋ₓ's are, therefore, present in the form of the above-described particles.

Preferred antigens according to the invention are in particular the HBcAg₁₋₁₂₄, HBcAg₁₋₁₂₇, the HBcAg₁₋₁₄₄, the HBcAg₁₋₁₄₅, the HBcAg₁₋₁₄₆, the HBcAg₁₋₁₄₇, the HBcAg₁₋₁₄₈ and the HBcAg₁₋₁₄₉, (i.e. polypeptides which comprise, preferably which consist of the first 127, 144, 145, 146, 147, 148 or 149 amino acids of the natural HBcAg) . A further preferred antigen is HBcAg_{1-144+AS}, a polypeptide which comprises the first 144 amino acids of the naturally occurring HBcAg's and which as 145^{th} amino acid comprises an amino acid AS which is different to the glutamic acid appearing as 145^{th} amino acid in the naturally occurring HBcAg, whereby this amino acid AS which is different to glutamic acid is preferably isoleucine. In the composition according to the invention, antigens which are particularly preferably comprise HBcAg₁₋₁₄₅ and HBcAg₁₋₁₄₄₊₁.

It is furthermore preferred according to the invention that the HBcAg₁₋ₓ does not comprise any epitopes which are foreign to HBcAg, in particular no HBsAg-epitopes. Most preferably, the HBcAg₁₋ₓ's comprise no pre-S1 epitopes, in particular not the amino acids 3 to 55 of the pre-S1 antigen. It is also preferred according to the invention that the HBcAg₁₋ₓ comprises no cysteine residue at the C-terminal position x, as long as x is not equal to 48, 61 or 107.

According to a particular embodiment of the composition according to the invention, this comprises at least two HBcAg₁₋ₓ's or variants thereof, whereby the HBcAg₁₋ₓ's differ in the HBV-genotype.

The composition according to the invention comprises an adjuvant comprising a saponin. This adjuvant is a saponin complex . Particularly preferably, the saponin complex is a liposomal saponin complex.

The scope of the present invention is defined by the claims and any information that does not fall within the claims is provided for information only

In this context it is particularly preferred that the saponin complex, comprises, as components which are different to water or aqueous salt solutions
(α1) 0 to 95 wt.%, particularly preferably 10 to 50 wt.% and most preferably 15 to 25 wt.% of a phospholipid,
(α2) 0 to 95 wt.%, particularly preferably 10 to 50 wt.% and most preferably 15 to 25 wt.% of a steroid,
(α3) 5 to 100 wt.%, particularly preferably 15 to 90 wt.% and most preferably 30 to 80 wt.% of the saponin or of the saponin derivative, and
(α4) 0 to 20 wt.%, particularly preferably 1 to 15 wt.% and most preferably 5 to 10 wt.% further additives, for example further lipids which are different to phospholipids and steroid lipids or solvents which are different to water,
whereby the weight amounts are respectively based on the total weight of the saponin complex, with the exception of its water portion or of its aqueous salt solutions portion, and the sum of the components (α1) to (α4) is 100 wt.%.

The saponin complexes of this type are also mentioned in the literature *inter alia* as "ISCO^{®}-Matrix". If the composition also comprises proteins as antigens in addition to the components (α1) to (α4), the complex is then referred to as "ISCOM^{®}".

The phospholipids (α1) are preferably phospholipids selected from the group consisting of phosphatidylcholine, phosphatidylethanolamine and dipalmitoylphos-phatidylcholine.

The steroids (α2) are preferably steroids of plant or animal origin selected from the group consisting of cholesterol, lanosterol, lumisterol, stigmasterol and sitosterol.

As saponins are understood according to the invention preferably chemical compounds between a sugar and a steroid, steroid alkaloid or triterpene or mixtures of these compounds. These can also be referred to as steroid-saponins, steroid alkaloid saponins and triterpene saponins. Such saponins are surface active glycosides and can be obtained from plants, whereby the saponins which are most effective as adjuvants are obtained from the South American tree *Quillaja saponaria*, preferably from the bark of this tree. During the isolation of the saponins from *Quillaja saponaria*, an extract is obtained which in the partially purified form is known as "Quil A" (the partially purified saponin extract has a defined triterpene saponin content and is less toxic than the crude saponin extract).

Further suitable saponins are described in R. Tschesche and Wulf, "Chemie und Biologie der Saponine in Fortschritte der Chemie Organischer Naturstoffe", published by H. Herz, H. Grisebach, G. W. Kirby, Vol. 30 (1973), in particular strong polar saponins such as the polar triterpene saponins, in particular the polar acetic bisdesmosides, for example the saponin extract from *Quillajabark Aralosid A*, Chikosetsusaponin IV, Calendula-Glycoside C, Chikosetsusaponin V, Achyrranthes-Saponin B, Calendula-Glycoside A, Aralosid B, Aralosid C, Putranji-Saponin III, Bersamasaponisid, Putrajia-Saponin IV, Trichosid A, Trichosid B, Saponasid A, Trichosid C, Gypsosid, Nutanosid, Dianthosid C, Saponasid D, preferably Aescin from *Aesculus hippocastanum* (T. Patt and W. Winkler: "Das therapeutisch wirksame Prinzip der Rosskastanie (Aesculus hippocastanum)", Arzneimittelforschung 10(4), 273-275 (1960)).

Saponin (α3) is preferably the partially purified saponin extract from the bark of *Quillaja saponaria* referred to as "Quil A", which consists of at least 22 different saponin fractions, sub-fractions of this homogenous Quil A-fraction, such as, for example, QS 7, QS 17, QS 18 or QS 21, or a mixture of at least two of the sub-fractions obtained from the homogenous Quil A-fraction, preferably a mixture of the sub-fractions A and C, as described in WO-A-96/11711. This mixture preferably consists of 50 to 90 wt.% of fraction A and 10 to 50 wt.% of fraction C, whereby the fractions A and C are obtained according to example 1 of WO-A-96/11711. Mixtures of this sort are commercially available, for example under the reference "ISCOPREP^{®}703". According to a particular embodiment, the saponin can also consist to 100 wt.% of fraction C. In addition to the saponin from Quil A, saponin derivatives, as described, for example, in US 6,262,029, can also be comprised in the complex. Suitable saponin complexes according to the invention are, in particular, the commercially obtainable products AbISCO^{®}-100, AbISCO^{®}-200, AbISCO^{®}-300 (ISCONOVA, Uppsala, Sweden) or Stimulon^{®}QS-21 (ANTIGENICS, Woburn, USA).

The production of the above-described saponin complexes (ISCO^{®}-matrix) can be carried out by any process for production of such complexes which is known to the skilled person. Preferred processes for production of saponin complexes of these types are, for example, described in EP-A-0 231 039 (liposomal saponin complexes) or in WO-A-90/03184 (non-liposomal saponin complexes).

If an ISCO-matrix is comprised in the composition according to the invention as adjuvant, it is further preferred according to the invention that the antigen or the antigens is or are not localised in the inside of the ISCO^{®}-matrix, as is the case with known ISCOMs.

According to a particular embodiment of the composition according to the invention, this can also comprise as co-adjuvants, in addition to the saponin complex, further adjuvants which do not comprise saponin or saponin derivative, whereby these co-adjuvants can be present separate from the complex or integrated into this complex. As co-adjuvant, any adjuvant known to the skilled person can be used which is commonly used in vaccines. Preferred adjuvants are selected from the group comprising particulate adjuvants, such as, for example, sub-micro oil-in-water-emulsion, to which belong, for example, MF59 (5 % Squalene, 0,5 % Tween^{®} 80 and 0,5 % Span^{®} 85), SAF (10 % Squalene, 0.4 % Tween^{®} 80, 5 % Pluron-blocked polymer) and thr-muramyl dipeptide (thr-MDP), mineral compounds such as, for example, aluminium hydroxide, aluminium sulphate or aluminium phosphate, incomplete Freund's adjuvant, lipopolysaccharides, N-acetylglucosaminyl-N-acetylmuramyl-L-alanyl-dipeptide (GMDP) or muramyldipeptide (MDP), GAL-ceramide, dimethyldioctadecylammonium bromide (DDAB), liposomes, preferably comprising phospholipid and cholesterol, microparticles of biodegradable polymers such as poly-lactide-co-glycolide (PLGA), oligodesoxyribonucleotides with or without CpG-motif, N,N-di-(β-stearoylethyl)-N,N-dimethylammonium chlorid (EQ1), glycopeptides, components of the cell wall of mycobacteria, quaternary amines such as, for example, cetylpyridinium chloride and benzalkonium chloride, zwitterionic amines such as CHAPS (3-(3-cholamidopropyl)-dimethyl-ammonio)-1-propanesulfonate), dextran sulfate, granulocyten macrophages colonies stimulating factor (GM-CSF), tumor necrosis factor (TNF), block copolymers such as, for example, P1205 or Poloxamer 401, dimyristoylphosphatidylcholine (DMTC), 3β-hydroxy-5-androsten-17-one (DHEA), dimyristoylphosphatidyl-glycerol (DMPG), desoxycholic acid sodium salt, Imiquimod, DTP-GDP, 7-allyl-8-oxoguanosine, Montanide ISA^{®} 51, Montanide ISA^{®} 720, murametide, murapalmitin, dicetylphosphate, polymethyl methacrylate (PMMA), PEG-sorbitan fatty acid esters such as polysorbate 20 or 80 (TWEEN^{®} 20, 80), detoxified mutants from bacterial ADP-ribosylating toxins such as cholera toxin or pertussis toxin or mixtures of at least two of these adjuvants. Particularly preferred as co-adjuvants are lipopolysaccharides, aluminium hydroxide, aluminium phosphate, GAL-ceramide, oligodesoxyribonucleotide with CpG-motif, as well as PEG-sorbitan fatty acid esters.

An overview of compounds suitable as co-adjuvants is presented in Cox, J.C. and Coulter, A.R. "Adjuvants - a classification and review of their modes of action" in Vaccine 15:248-256 (Feb. 1997).

The composition according to the invention further comprises, in addition to the antigen HBcAg₁₋ₓ and the adjuvant at least one HBsAg, or a variant of this antigen . In this context it is particularly preferred that not only the HBcAg₁₋ₓ but also the HBsAg is present in particulate form in the composition according to the invention.

The HBsAg is a 226-amino acid protein (p24/gp27 or S-protein), which self assembles in 20-30 nm lipoprotein particles, in which around 100-150 sub-units are cross-networked by means of multiple inter-and intra-molecular disulfide links. The HBsAg's optionally comprised in the composition according to the invention can be derived from all 8 HBV-standard genotypes A, B, C, D, E, F, G and H known at this time, in the same way as the HBcAg₁₋ₓ's. The term "HBsAg" also further comprises, in addition to 226-amino acid protein, both M- and L-proteins, which, in addition to the HBsAg also comprise the 55-amino acid long pre-S2-peptide (M-protein) or the 55-amino acid long pre-S2-peptide and the 120-amino acid long pre-S1-peptide (L-protein).

By the term "variants" of the HBsAg or of the fragment of the HBsAg is understood a polypeptide in which at most 5, preferably at most 2 and particularly preferably at most 1 amino acid of HBsAg is deleted, inserted, appended at the ends or substituted, preferably substituted.

According to preferred embodiment of the composition according to the invention, this comprises not only the HBsAg, but, as described in WO-A-2005/02397, at least two HBsAg's or fragments or variants thereof, whereby the HBsAg's differ in the HBV-genotype in the S-region and/or in the pre-S1-region. In this context it is particularly preferred that the two HBsAg's which differ in the HBV-genotype are present in the form of homologous particles, as described likewise in WO-A-2005/02397. By a homologous HBsAg particle is understood a particle which is composed solely of HBsAg of the same HBV-genotype.

The HBsAg's can be obtained by processes known to the skilled person, either from the serum of patients with chronic hepatitis B, synthetically or as recombinant polypeptides. In this context, reference is made to the above-described methods in connection with the obtaining of HBcAg₁₋ₓ.

The composition according to the invention can also comprise further additives iv), such as pharmaceuticals carriers or additives, in addition to the components i), ii) and iii), in particular if it is present as pharmaceutical formulation.

Preferred pharmaceutical carriers are in particular water, buffered water, preferably isotonic salt solutions such as PBS (Phosphate Buffered Saline), glucoses, dextroses, mannitol, lactoses, starches, magnesium stearate, celluloses, magnesium carbonate, 0.3 % glycerol, hyaluronic acid, ethanol, polyalkylene glycols such as polypropylene glycol, triglycerides or the like. The type of pharmaceutical carrier used is particularly dependent on whether the composition according to the invention is formulated for oral, nasal, intradermal, subcutaneous, intramuscular or intravenous administration. As additives, tonicity regulators, wetting agents, emulsifies or buffer substances can be comprised in the composition according to the invention.

The relative weight ratios between the antigen components i) and iii) to adjuvant ii) preferably lies within a range from 1 : 20 to 20: 1, particularly preferably within a range from 1 : 10 to 10 : 1, yet more preferably within a range from 1 : 5 to 5 : 1 and most preferably within a range from 1 : 2 to 2 : 1. The weight ratio between adjuvant and antigen which is optimal for the respective application case can be determined by the skilled person by means of simple trials, in which, for example, the strength of the CTL-response is determined dependent on this weight ratio.

The weight ratio between the HBcAg₁₋ₓ and the HBsAg preferably lies within a range from 1 : 20 to 20 : 1, particularly preferably within a range from 1 : 10 to 10 : 1, yet more preferably within a range from 1 : 5 to 5 : 1 and most preferably within a range from 1 : 2 to 2 : 1. The skilled person can also determine the optimal weight ratio here by means of simple trials.

The total concentration of the components i), ii) and iii) in the composition according to the invention (i.e. the total amount of the components i), ii) and iii) based on the volume of the composition according to the invention) preferably lies within a range from 0.1 to 2,000 µg/ml, particularly preferably within a range from 0.5 to 1,500 µg/ml, yet more preferably within a range from 1 to 1,000 µg/ml and most preferably within a range from 10 to 500 µg/ml, as long as the composition according to the invention is not present as lyophilisate.

According to an embodiment of the composition according to the invention the invention, this comprises
i) an HBcAg₁₋₁₄₅,
ii) a saponin complex as adjuvant, preferably a liposomal saponin complex, and
iii) an HBsAg.

According to a further embodiment of the composition according to the invention, this comprises
i) an HBcAg₁₋₁₄₄₊₁,
ii) a saponin complex as adjuvant, preferably a liposomal complex, and
iii) an HBsAg.

The composition according to the invention is preferably further characterised in that is has an HBcAg-specific CD8⁺ T-cell frequency in the spleen of at least 30, particularly preferably at least 50, yet more preferably at least 75, even more preferably at least 100 and most preferably at least 300 CD8⁺ IFNγ⁺ T-cells per 10⁵CD8⁺ T-cells in C57BL/6-mice with an immunization according to the herein-described immunization process and subsequent re-stimulation described herein with MGLKFRQL (a synthetic fragment of HBcAg's).

The composition according to the invention is further preferably characterised in that, it induces in C57BL/6-mice potent antibody responses against HBsAg with an immunization according to the herein-described immunization process. The total anti-HBs antibody count, determined according to the herein-described AUSAB^{®}-assay at up to, on average, at least 100 mIU/mL, preferably at least 500 mIU/mL, particularly preferably at least 1,000 mIU/mL and most preferably at least 1,500 mIU/mL. The determination of the anti-HBs-specific IgG-isotype-class shows that both IgG1- and IgG2b-counts are increased, which is an indication of the formation of both a Th1 and a Th2 immune response.

A further contribution to the solution of the above-mentioned object is provided by a process for production of a composition, comprising the process steps I), In and IV) and III):
I) provision of a HBcAg₁₋ₓ, whereby x is a whole number from the range from 100 to 160, preferably the range from 120 to 150, particularly preferably the range from 140 to 149, yet more preferably the range from 144 to 146 and most preferably is equal to 145, or of a variant of this antigen,
II) provision of an adjuvant comprising a saponin, wherein the adjuvant is a saponin complex,
III) provision of a HBsAg, or of a variant of this antigen , and
IV) bringing into contact of the HBcAg₁₋ₓ, of the adjuvant and of the HBsAg.

As HBcAg₁₋ₓ or variant thereof, as adjuvant comprising a saponin, as well as as HBsAg or as variant thereof, those compounds which were already mentioned as preferred components i), ii) and iii) in connection with the composition according to the invention are preferred.

With respect to the process steps I) and III), it is preferred that the provision of the HBcAg and of the HBsAg occurs in such a way that the antigens obtained from the plasma of patients with chronic hepatitis B (only in the case of HBsAg) by synthetic processes or by recombinant expression are presented in a suitable pharmaceutical carrier. This pharmaceutical carrier is preferably a liquid, particularly preferably an aqueous liquid, yet more preferably an aqueous salt solution, such as PBS. Before the bringing into contact with the other components in process step IV), the aqueous antigen solutions obtained in this way can be sterile filtered by processes known to the skilled person.

It is preferred according to a preferred embodiment of the process according to the invention that the provision of the HBcAg and of HBsAg in process steps I) and III) occurs in such a way that these antigens are first presented in a pharmaceutical carrier, preferably in an aqueous salt solution, in a concentration which is at least double, particularly preferably at least three times, yet more preferably at least four times and most preferably at least six times as great as the concentration of the HBcAg or of the HBsAg in the final targeted composition according to the invention (thus, should, for example, the concentration of HBcAg in the composition according to the invention amount to 10 µg/ml, it is preferred in the production of the composition that the HBcAg is first provided in an aqueous salt solution in a concentration of at least 20 µg/ml, particularly preferably at least 30 µg/ml, yet more preferably at least 40 µg/ml and most preferably at least 60 µg/ml). It is preferred to present HBcAg and HBsAg together in one and the same salt solution in the above-mentioned concentrations (at least two times, at least three times, at least four times or at least six times over-concentrated). It is further preferred according to the invention that these over-concentrated antigen-solutions, before the bringing into contact with the adjuvant in process step IV), are incubated for a time period of 10 minutes to 4 hours, particularly preferably from 20 minutes to 2 hours and yet more preferably from 30 minutes to 1 hour at a temperature within a range from 30 to 60 °C, particularly preferably 35 to 55 °C and yet more preferably 37 to 50 °C. After the bringing into contact of the antigen with the adjuvant, it is furthermore preferred that the thus-obtained composition is incubated for a time period from 15 minutes to 5 hours, particularly preferably from 30 minutes to 3 hours and yet more preferably from 45 minutes to 2 hours at a temperature from 10 to 40 °C, particularly preferably from 15 to 30 °C, yet more preferably from 20 to 25 °C and most preferably at room temperature.

In process step II) the adjuvant is provided. It is also here preferred that the adjuvant, before being brought into contact with components I) or III), is first sterilised, for example by sterile filtration, as long as the adjuvant used is not already commercially obtainable in sterile form.

In particular, if complexes from saponin, steroids and/or phospholipids are used as adjuvant (ISCO^{®}-matrix), it is preferred according to the invention that, first, the complexes are prepared in sterile form or obtained commercially from appropriate suppliers and then the saponin complex or the saponin derivatives complex is brought into contact with the sterilised, aqueous HBcAg solution and with the sterilised HBsAg solution or with a mixture of these two solutions. In this way, the formation of a classic ISCOM-complex, in which the antigens are incorporated into the interior of the ISCO^{®}-matrix (so-called ISCOMs) does not occur.

The thus-obtained compositions can then be further combined with further additives, such as, for example, further pharmaceutical carriers and additives, depending on whether the composition should be administered orally, intranasally, intradermally, subcutaneously, intramuscularly or intravenously. As pharmaceutical carrier and additive, those compounds are in turn preferred which have already been mentioned as preferred pharmaceutical carriers and preferred additives in connection with the composition according to the invention.

If aqueous solutions are obtained, these can be packaged or lyophilised in aqueous, sterile state, whereby the lyophilised composition is combined with a sterile solution before administration.

A further contribution to the solution of the above-mentioned object is provided by the compositions obtainable by the above-described process, whereby these compositions preferably have the same properties as the above-described composition according to the invention.

A contribution to the solution of the above-mentioned objects is also provided by a pharmaceutical formulation comprising the composition according to the invention as well as at least one of the above-mentioned additives iv).

A contribution to the solution of the above-mentioned objects is also provided by the use of the composition according to the invention or of the pharmaceutical formulation according to the invention for therapy and/or for prophylaxes of HBV-infections and HBV-mediated diseases. Preferably, the composition according to the invention or the pharmaceutical formulation according to the invention is used for administration in mammals, in particular human. According to a preferred use of the composition according to the invention or of the pharmaceutical formulation according to the invention, this serves in particular for therapy and/or for prophylaxes, preferably for therapy, of acute and/or chronic, preferably chronic HBV-infections.

The term "prophylaxis" in the context of an HBV-infection in humans comprises preferably
- the treatment of a human who has not yet any contact with HBV, with the aim of preventing the occurrence of an infection with HBV,
- the treatment of a human who has already had contact with HBV and who is in the immunotolerant chronic phase, with the aim of preventing this human from entering into the immunoactive chronic phase, as well as
- the treatment of a human who has already had contact with HBV and which is in the inactive carrier stage, with the aim of preventing this person from newly entering into the immunoactive phase.

The term "therapy" in connection with a HBV-infection in humans comprises preferably
- the treatment of a human who has already had contact with HBV and who is in the acute phase of the infection,
- the treatment of a human who has already had contact with HBV and who is in the immunoactive chronic phase.

Particularly preferably, the composition according to the invention or the pharmaceutical formulation according to the invention is administered to patients who are already infected with HBV.

People in the acute infection phase, in the immunotolerant chronic phase, in the immunoactive chronic phase or in the inactive carrier stage can be treated with the composition according to the invention or with the pharmaceutical formulation according to the invention, separately or in combination with other therapies as necessary. In therapeutic applications, the composition or the pharmaceutical formulation respectively is administered to the patient in an amount which is sufficient in order to cause an effective CTL-response against HBV antigens and heals or at least partially stops HBV-associated symptoms and/or complications. If respectively the composition according to the invention or the pharmaceutical formulation according to the invention, as particularly preferred, is used for therapy of chronic hepatitis B, the aim of the therapy is a reduction or, in the ideal case, an elimination of virus-infected cells. Since people can develop a chronic hepatitis B because of an inadequate or lacking CTL-response during the acute phase of their infection, it is important to provide an amount of the immune-relevant viral antigens (HBcAg₁₋ₓ and HBsAg) in a composition according to the invention or in the pharmaceutical formulation according to the invention respectively, which is sufficient in order to effectively stimulate a CTL-response. Amounts with which these aims are achieved are defined as "therapeutically effective doses". The dose which is therapeutically effective for the respective application case depends, for example, on the exact composition of the composition according to the invention or of the pharmaceutical formulation according to the invention respectively, on the method of administration, on the stage and the severity of the treated disease, on the weight and general state of health of the patient as well as on the judgement of the prescribing doctor, but generally lies preferably within a range from around 0.1 to 2,000 µg (total amount of adjuvants, HBcAg₁₋ₓ and HBsAg) for a patient weighing 70 kg, particularly preferably within a range from 0.5 to 1,500 µg, yet more preferably within a range from 1 to 1,000 µg and most preferably within a range from 10 to 500 µg, followed by renewed doses within a range from 0.1 to 2,000 µg over weeks to months, depending on the CTL-response of a patient, which is determined by measurement of HBV-specific CTL-activity in the peripheral blood lymphocytes (PBL) of the patient.

In the therapy of chronic hepatitis B, the administration should be continued for a length of time until at least the clinic symptoms or the laboratory indicators indicate that the HBV-infection has been eliminated or is at least under the control of the immune system, optionally for longer.

The composition according to the invention or the pharmaceutical formulation according to the invention can, in principal, be administered orally, intranasally, intradermally, subcutaneously, intramuscularly or intravenously.

A contribution to the solution of the above-mentioned object is, furthermore, provided by the use of the composition according to the invention for production of a pharmaceutical formulation for therapy and/or prophylaxis of HHV-infections and HBV-mediated diseases.

In this context, a particular contribution to the solution of the above-mentioned object is provided by the use of the composition according to the invention, comprising
i) the above-described HBcAg₁₋ₓ,
ii) the above-described adjuvant comprising a saponin, as well as
iii) the above-described HBsAg,
for production of a pharmaceutical formulation for therapy of HBV-infections and HBV-mediated diseases, preferably for therapy of HBV-infections and HBV-mediated diseases, whereby the HBV-infections or the HBV-mediated disease has been caused by an HB-virus whose genotype or subtype, preferably genotype, differs from the genotype or subtype, preferably genotype, of the HBsAg comprised in the composition according to the invention. If, for example, the HBV-infection or the HBV-mediated disease is caused by an HB-virus of the genotype A, for the therapy of this infection of this disease, preferably a composition according to the invention is used which uses, in addition to components i) and ii), an HBsAg of the genotype B, C, D, E, F, G or mixtures of HBsAg of these genotypes, whereby an HBsAg of genotype A can also be comprised.

Furthermore, a contribution to the solution of the above-mentioned objects is made by a process for therapy and/or for prophylaxis of HBV infections and HBV-mediated diseases, in which a human who has not yet come into contact with HBV or a human who has already come into contact with HBV, preferably a patient suffering from chronic hepatitis B, is administered a therapeutically effected dose of the composition according to the invention or of the pharmaceutical formulation according to the invention, preferably in the above-described art and way.

It can also be particularly advantageous here to administer to the person who has already come into contact with HBV, preferably to the patient suffering from chronic hepatitis B, such a composition which also comprises, in addition to the HBcAg₁₋ₓ and the adjuvant, an HBsAg which is derived from a HBV-genotype or HBV-subtype, which differs from the genotype or subtype, preferably genotype, of the HB-virus which is causal for the HBV-infection or for the HBV-mediated disease of the person to be treated.

The invention is now more closely illustrated by means of non-limiting figures and examples.
Figure 1 shows the CTL-responses when using compositions which is not according to the invention with AbISCO^{®-}100 as adjuvant.
Figure 2 shows the CTL-responses when using compositions according to the invention with AbISCO^{®-}200 as adjuvant.
Figure 3 shows the B-cell-response when using compositions according to the invention which comprise HBcAg and HBsAg, with AbISCO^{®}-200 as adjuvant, illustrated by the AUSAB-anti-HBsAg count.
Figure 4 shows the immune profile of the B-cell-response when using compositions according to the invention which comprise HBcAg and HBsAg, with ABISCO^{®}-200 as adjuvant, illustrated by the count of anti-HBsAg-IgG1 and anti-HBsAg-IgG2b isotype. The composition according to the invention induces both a Th1 and a Th2 immune response.

### METHODS

### Immunization of C57BL/6 mice

C57B1/6-mice were immunized with PBS, HBcAg, HBsAg and adjuvant in the amounts given in tables 1 and 2 in respectively 100 µl PBS, whereby the respectively given amounts were respectively injected subcutaneously twice at a separation of 21 days. For the DNA immunization, the plasmid pCI/HBV_{ayw} core was injected in amounts of 2×50 µg in respectively 50 µl PBS into each *Tibialis anterior* muscle.

### Determination of the antigen-specific CD8⁺ T-cell-frequency in the spleen

Two weeks after the second immunization, the spleen was removed from the animals and cell suspensions produced from the spleen cells. The production of spleen cell suspensions is described in Schirmbeck R., Böhm W., Fissolo N., Melber K., and Reimann J.: "Different imunogenicity of H-2 Kb-restricted epitopes in natural variants of the hepatitis B surface antigen ", Europ. J. Immunol. 2003, 33: 2429-38. The spleen cells were incubated (re-stimulation) for four hours in RPMI-1640 medium with 0.25 µM HBcAg-specific peptide (MGLKFRQL; Jerini BioTools, Berlin, DE) and 5 µg/ml Brefeldin A (BFA) (catalogue no. 15870, Sigma) at 37 °C. The re-stimulated cells were harvested and the surface was colored with anti-CD8 mAb (anti-mouse CD8a-PE-conjugate; catalogue no. 55303; BD Biosciences, Heidelberg, DE) fixed, permeabilised and a coloration on cyto-plasmatic IFNγ (anti-mouse IFNγ-FITC-conjugate; catalogue no. 554411; BD-Biosciences, Heidelberg, DE) carried out. The frequencies of core-specific CD8⁺IFNγ⁺CTLs were determined by FACS analysis. The average value for CD8⁺IFNγ⁺T-cells/10⁵ CD8⁺T-cells is shown.

### Determination of the antibody-isotype

96-well-microtiter plates (Nunc, Wiesbaden, DE) were coated with the HBsAg (2 µg/mL in carbonate buffer) used for immunization overnight at 4 °C (100 µL per well.

At the start of the ELISA-process, the coated plates were washed three times with PBST (phosphate buffered salt solution with 0.5 % Tween 20). The wells were blocked with 0.2 % gelatine for 1 hour at 37 °C. The mouse plasmas/serums to be determined were diluted in two steps in PBS (within the range 1:250 to 1:32,000) and respectively 100 µL of the dilutions added to the appropriate wells (in duplicate) and incubated for 1 hour at 37 °C. Serum from non-immunised mice was used as control. The microtiter plate was then washed five times with PBST and incubated with an isotype-specific second antibody (peroxidase-conjugated). To test for IgG1, gamIg1 (catalogue no. GAM/IgG1/PO) was used and to test for IgG2b, gamIg2b (catalogue no. IgG2b/PO; Nordic Immunological Laboratories, Tilburg, Netherlands) was used. The colour reaction was then carried out with o-phenylenediamine (oPD) (Sigma; catalogue no. P2903) as substrate (1 mg/ml oPD with 1 µL/mL 30 % H₂O₂) (100 µL per well). The colour reaction was stopped after 10-15 minutes by means of 1 N H₂SO₄ (50 µL per well) and the optical density at 492 nm measured in the spectrophotometer (SpectraMax plus; Molecular Devices, Ismaning, DE).

### Determination of the total anti-HBs antibody count

The anti-HBs antibody count in the serums of the immunized mice was determined in the IMX Automated Immunoassay Analyzer (Abbott Diagnostics, Wiesbaden, Germany) using the IMX AUSAB^{®} test kit (reference no. 2226-21, Abbott, Wiesbaden, Germany). The assay was carried out as described by the manufacturer.

### Preparation of the antigens

### a) HBcAg_{1-144+I}

By means of BamHI-restriction, the plasmid pHB320 (7,543 base pairs), which carriers the complete HBV genome, was constructed from the vector pBR322 (Bolivar F., Rodriguez R.L., Greene P.J., Betlach M.C., Heyneker H.L., Boyer H.W.: "Construction and Characterization of new cloning vehicles. II. A multipurpose Cloning System," Gene. 1977; 2(2):95-113) and the circularised hepatitis B virus genome (HBV320, 3,182 base pairs; Bichko V, Pushko P, Dreilina D, Pumpens P, Gren E (1985), "Subtype ayw variant of hepatitis B virus", FEBS 185: 208-212).

After *Hha*I-restriction and S1-nuclease decomposition of the overhanging individual strand sequences, a 1,000 base pair long fragment, which carries the gene sequences for the pre-core/core-gene (*core open reading frame*) was cloned by means of *Blunt*-*end*-ligation into the vector pBR322 P*trp* (Ikehara M, Ohtsuka E, Tokunaga T. et al. (1984): Synthesis of a gene for human growth hormone and its expression in E. coli.; Proc. Natl. Acad. Sci. USA 81:5956-5960). This vector had been cut beforehand with *Bam*HI/*Sal*I and the projecting ends filled with DNA polymerase. The resulting plasmid pGC74 (6,046 base pairs) carries the tryptophan promoter and the HBc-gene in opposing orientation. This orientation facilitates the following cloning steps by the reconstitution of singular restriction sites for optimisation of the HBcAg-expression cassette.

To change the orientation of the HBc-fragements, pGC74 was restricted with *Bam*HI, the projecting ends filled with DNA-polymerase and post-restricted with *Sal*I. This fragment was ligated with the vector pBR322 P*trp*. This vector had been linearized beforehand with *Ava*I, the ends filled with DNA-polymerase and post-restricted with *Sal*I. The thus-constructed plasmid pGC2 (5,552 base pairs) carries the tryptophan promoter and the HBc-gene in the same orientation.

This plasmid was restricted with *Sal*I, the projecting ends removed with the nuclease *Bal*31, post-restricted with *Eco*RI, the thus-resulting overhanging ends filled with DNA-polymerase and ligated again. Plasmid pHBc10 (4,825 base pairs) resulted, with a shortened sequence between tryptophan-promotor and HBc-gene to obtain an optimal P*trp*-Shine-Dalgamo-HBc structure or distance.

For optimization of the gene expression, a 1,044 base pair-*Hha*I-HBc-fragment was digested from pHBc10 after restriction with *Hhal*I with S1-nuclease and cloned into the vector pBR322-*trp*-I, a modified pBR322-plasmid. This construction resulted in pHBc3 (7,108 base pairs), with the optimised expression sequence of the HBc-gene under the control of the tryptophan promoter.

The plasmid pHBc3 was restricted with *Kpn*21 and a synthetic polylinker *Eco*RV-*Cla*I-*Pvu*I (5'TCCGGAGATATCGATCGTCCGGA-3') incorporated into the restriction site at the amino acid position 144 of the HBc gene. Plasmid pHBc1615 (7,129 base pairs) resulted therefrom. PHBc1615 was linearised with *Cla*I and a synthetic polylinker with a termination codon after amino acid position 144 and with an additional isoleucine codon at position 145 incorporated. Plasmid pHBc2-7 was obtained.

Competent *E*. *coli* cells (Stem BL21: Genotype B F⁻dcm ompT hsdS (r_{B}⁻m_{B}⁻)gal) were transformed with plasmid pHBc2-7 and obtained after plating on LB Agar with ampicillin overnight at 37 °C. With respectively one individual colony, 300 ml M9 minimal medium (with addition of 1 % Casamino acids, Difco catalogue no. 223050, Becton-Dickinson, Heidelberg) and 0.2 % glucose are inoculated. The cultivation occurred overnight at 37°C on a round shaker in 1 I-Erlenmeyer flask. The cultures generally reached an optical density (OD₅₄₀ₙₘ) between 2 and 5.

The cells were centrifuged and the pellet re-suspended in lysis buffer (50 mM TRIS/HCI pH 8.0; 5 mM EDTA, 100 µg/ml PMSF, 0.08 % Triton-X-100). The conditioned cells were then lysed on ice by means of ultrasound treatment (3 x 15 seconds at 22 kHz). After centrifugation of the cell debris, the proteins were precipitated from the supernatant with ammonium sulphate (33 % saturation) for 4-20 hours at 4 °C. The pellet was re-suspended in PBS with 0.1 % Triton-X-100 and then respectively 5-10 ml of the obtained solution charged onto a Sepharose CL4B-chromatography column (2.5×85 cm). The elution occurred with PBS without addition of Triton-X-100. The presence of HBcAg-polypeptides in the individual fractions was investigated by means of polyacrylamide-gel-electrophoresis / Coomassie-coloration. Positive fractions were purified and concentrated by means of ammonium sulphate precipitation (33 % saturation) for 20 hours at 4 °C. The protein pellets were re-suspended in PBS with a concentration between 3-20 mg/ml and then dialysed overnight against 1,000 volumes of the same buffer. The HBcAg_{1-144+I} was sterile filtered in PBS and stored at -20 °C.

### b) HBsAg

HBsAG was prepared as described in: Brocke P, Schaefer S., Melber K., Jenzelewski V., Müller F., Dahlems U., Bartelsen O., Park K.N., Janowicz Z.A., Gellissen G.: "Recombinant hepatitis B vaccines: disease characterization and vaccine production" (2005) in Gellissen G (Editor): "Production of recombinant proteins - novel microbial and eucaryontic expression systems", pages 319-360, Wiley-VCH, Weinheim) and can be obtained from Rhein Biotech, Düsseldorf, Germany.

### c) HBcAg₁₋₁₈₃

HBcAG₁₋₁₈₃ was obtained from DiaSorin S.r.l. (Saluggia, Italy).

### EXAMPLE 1

In a first series of experiments, the saponin complex which is commercially obtainable under the product description AbISCO^{®}-100 (ISCONOVA, Uppsala, Sweden) was used as adjuvant. Core-plasmid-DNA was used as positive control, pure PBS, pure adjuvant in PBS, pure HBc₁₋₁₄₄₊₁ in PBS as well asHBc₁₋₁₈₃ in PBS as negative control. The composition according to the invention comprised PBS, HBc_{1-144+I} as well as AbISCO^{®}-100. The amounts of the individual components are given in table 1.

The plasmid pCI/HBV_{ayw} core was prepared as described in Kuhröber A., Pudollek H.P., Reifenberg K., Chisari F.V., Schlicht H.J., Reimann J., Schirmbeck R. (1996): "DNA Immunization induces antibody and cytotoxic T cell responses to hepatitis B core antigen in H-2b mice", J. Immunol. 156: 3687-95, (the construct is referred to there as pCMV-1/c) and isolated with QIAGEN-tip 500 (catalogue no. 12162; Qiagen, Hilden, Germany) as described by the manufacturer.

For the formulation of the composition, the HBc_{1-144+I} was first diluted with PBS to four times the concentration of the final product and incubated for 30 minutes at 37 °C with shaking. AbISCO^{®}-100, as delivered by the producer as aqueous dispersion, was then added and the end concentration of the antigen as given in table 1 was adjusted with PBS, whereby the concentration given in the table for the adjuvant and the antigen resulted. The thus-prepared composition was incubated for an hour at room temperature (20-25 °C), then stored overnight (12-18 hours) at 2-8 °C and used the following day for immunisation.

**Table 1**

| trial group | Composition used for immunisation |
|---|---|
| A (n. inv.¹⁾) | 2 × 50 µg core-Plasmid-DNA in 50 µl PBS (positive control) |
| B (n. inv.) | 100 µl PBS (negative control) |
| C (n. inv.) | 12 µg AbISCO^{®}-100 in 100 µl PBS (negative control) |
| D (n. inv.) | 10 µg HBcAg_{1-144+I} in 100 µl PBS (negative control) |
| E n. inv. | 12 µg AbISCO^{®}-100 and 10 µg HBcAg_{1-144+I} in 100 µl PBS |

| | |
|---|---|
| ¹⁾ n. inv. = non inventive | |

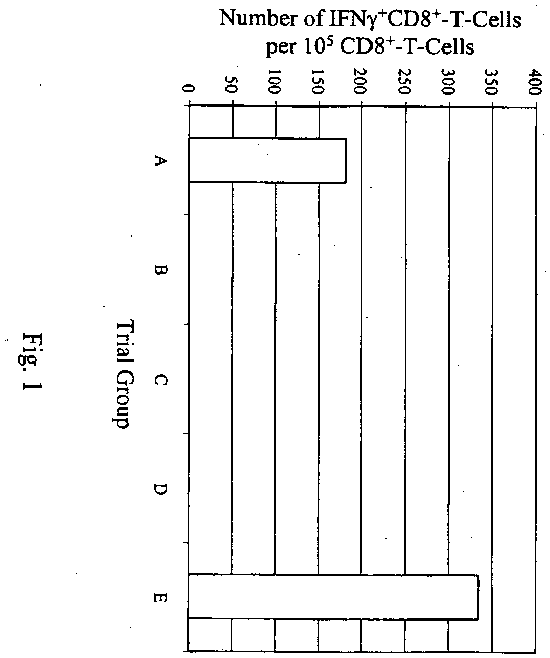

The CTL-responses achieved by the above-described composition can be found in figure 1.

### EXAMPLE 2

In a further series of experiments, the saponin complex which is commercially obtainable under the product description AbISCO^{®}-200 (ISCONOVA, Uppsala, Sweden) was used as adjuvant. As positive control, core-plasmid-DNA was used; as negative control pure PBS, pure adjuvant in PBS, pure HBc_{1-144+I} in PBS and pure HBc₁₋₁₈₃ in PBS. The composition according to the invention comprised PBS, HBc_{1-144+I} and AbISCO^{®}-200 and HBsAg. The amounts of the individual components are given in table 2.

For the formulation of the composition according to the invention, the HBc_{1-144+I}, and the HBsAg was first diluted with PBS to four times the concentration of the final product and incubated for 30 minutes at 37 °C with shaking. When using HBsAg, first, HBsAg and HBcAg_{1-144+I} were combined under aseptic condition and likewise diluted with PBS to four times the concentration of the final product. AbISCO^{®}-200 in the form of aqueous dispersion as delivered by the manufacturer, was then added and the end concentration of the antigen as given in table 2 was adjusted with PBS, whereby the concentration given in the table for the adjuvant and for the antigen or the antigens resulted. The thus-produced composition was incubated for one hour at room temperature (20-25 °C), then stored overnight (12-18 hours) at 2-8 °C and used the following day for immunization.

**Table 2**

| trial group | Composition used for immunisation |
|---|---|
| F (n. inv.) | 2 × 50 µg core-Plasmid-DNA in 50 ul PBS (positive control) |
| G (n. inv.) | 100 µl PBS (negative control) |
| H (n. inv.) | 5 µg AbISCO^{®}-200 in 100 µl PBS (negative control) |
| I (n. inv.) | 10 µg HBcAg_{1-144+I} in 100 µl PBS (negative control) |
| (n. inv.) | 10 µg HBcAg_{1-144+I} and 5 µg HBsAg in 100 µl PBS (negative control) |
| K (n. inv.) | 10 µg HBcAg₁₋₁₈₃ in 100 µl PBS (negative control) |
| L (n. inv.) | 5 µg AbISCO^{®}-200 and 10 µg HBcAg_{1-144+I} in 100 µl PBS |
| M (inv.) | 5 µg AbISCO^{®}-200, 10 µg HBcAg_{1-144+I} and 5 µg HBsAg in 100 µl PBS |

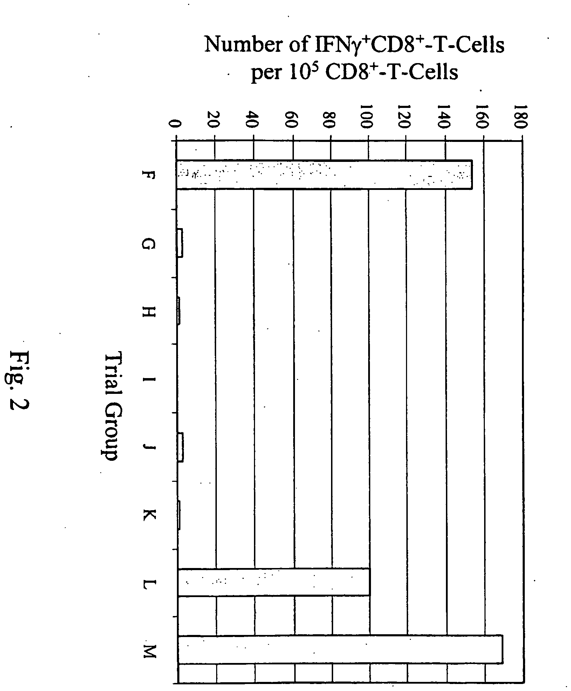

The CTL-responses achieved by the above-described compositions can be taken from figure 2. It can be seen from this figure that by means of the composition according to the invention (trial group M) core-specific CTL-responses can be achieved which are comparable with the CTL-responses which can be achieved with core-plasmid-DNA (trial group F).

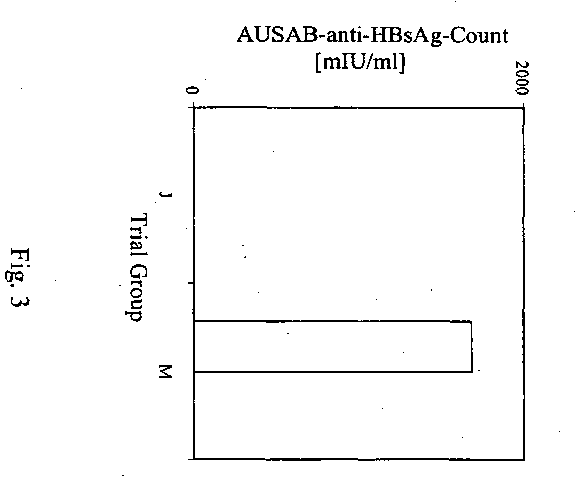

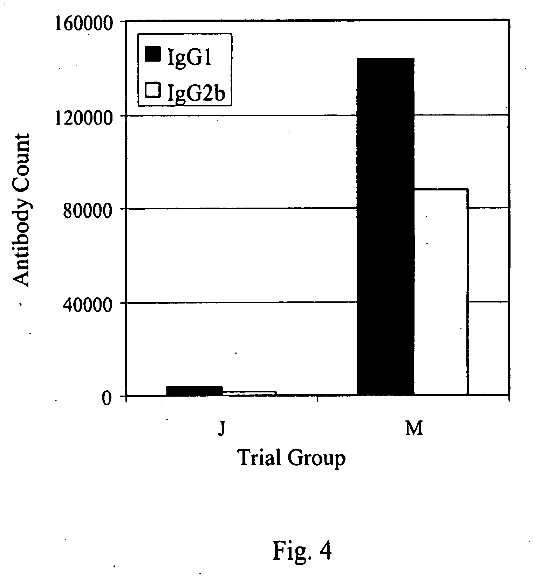

Figure 3 shows that the composition according the invention, as long as its also comprises HBsAg, also leads to a potent antibody response against HBsAg. Figure 4 shows in turn that by means of such a composition according to the invention, both IgG1- as well as IgG2b-counts are increased, which is indicative of the formation of both a Th1 and a Th2 immune response.

## Claims

1. A composition, comprising:
i) a HBcAg₁₋ₓ (HBcAg = Hepatitis B core antigen), wherein x is a whole number from the range from 100 to 160, or a variant of this antigen, wherein at most 5 amino acids of the HBcAg₁₋ₓ have been deleted, inserted, substituted or appended at the C- and/or N-terminal ends,
ii) an adjuvant comprising a saponin, wherein the adjuvant is a saponin complex, and
iii) a HBsAg (HBsAg = Hepatitis B surface antigen) or a variant of this antigen, wherein at most 5 amino acids of the HBsAg have been deleted, inserted, substituted or appended at the ends.

2. The composition according to claim 1, wherein x is a whole number from the range from 140 to 149.

3. The composition according to claim 1 or claim 2, wherein x is a whole number from the range from 144 to 146.

4. The composition according to any one of the preceding claims, wherein the adjuvant comprises as components which are different to water or aqueous salt solutions
(α1) 0 to 95 wt.% of a phospholipid,
(α2) 0 to 95 wt.% of a steroid,
(α3) 5 to 100 wt.% of the saponin or the saponin derivative, and
(α4) 0 to 20 wt.% further additives,
wherein the amounts by weight are based respectively on the total weight of the saponin complex, with the exception of its water portion or its aqueous salt solutions portion and the sum of the components (α1) to (a4) is 100 wt.%.

5. The composition according to any one of the preceding claims, wherein the relative amount ratio between the antigen components i) and iii) to adjuvant ii) lies within a range from 1 : 20 to 20 : 1.

6. The composition according to any one of the preceding claims, wherein the total concentration of the components i), ii) and iii) in the composition lies within a range from 0.1 to 2,000 µg/ml.

7. The composition according to claim 1, comprising
i) a HBcAg₁₋₁₄₅,
ii) a saponin complex, and
iii) a HBsAg.

8. The composition according to claim 1, comprising
i) a HBcAg_{1-144+I,}
ii) a saponin complex, and
iii) a HBsAg.

9. A process for preparation of a composition, comprising the process steps I)-IV):
I) provision of a HBcAg₁₋ₓ, wherein x is a whole number from the range from 100 to 160, or of a variant of this antigen, wherein at most 5 amino acids of the HBcAg₁₋ₓ have been deleted, inserted, substituted or appended at the C- and/or N-terminal ends,
II) provision of an adjuvant comprising a saponin, wherein the adjuvant is a saponin complex,
III) provision of a HBsAg or of a variant of this antigen, wherein at most 5 amino acids of the HBsAg have been deleted, inserted, substituted or appended at the ends, and
IV) bringing into contact of the HBcAg₁₋ₓ, of the adjuvant and of the HBsAg.

10. The process according to claim 9, wherein x is a whole number from the range from 140 to 149.

11. The process according to claim 9 or 1 0, wherein x is a whole number from the range from 144 to 146.

12. The process according to any one of claims 9 to 11, wherein the adjuvant comprises, as components which are different to water or aqueous salt solutions
(α1) 0 to 95 wt.% of a phospholipid,
(α2) 0 to 95 wt.% of a steroid,
(α3) 5 to 100 wt.% of the saponin or the saponin derivative, and
(α4) 0 to 20 wt.% further additives,
wherein the amounts by weight are based respectively on the total weight of the saponin complex, with the exception of its water portion or its aqueous salt solutions portion and the sum of the components (α1) to (α4) is 100 wt.%.

13. A composition obtainable by a process according to any one of claims 9 to 12.

14. A pharmaceutical formulation, comprising the composition according to any one of claims 1 to 8 or 13 and suitable additives.

15. The use of the composition according to any one of claims 1 to 8 or 13 for production of a pharmaceutical formulation for therapy and/or for prophylaxis of HBV infections and HBV-mediated diseases.

16. The use of the composition according to any one of claims 1 to 8 or 13 for preparation of a pharmaceutical formulation for treatment of chronic HBV infections.

17. The use according to claim 15 or 16, wherein the HBV infection or the HBV-mediated disease has been caused by a HB virus whose genotype or subtype differs from the genotype or subtype of the HBsAg comprised in the composition.

## Patentansprüche

1. Eine Zusammensetzung, beinhaltend:
i) ein HBcAg₁₋ₓ (HBcAg = Hepatitis B core antigen), wobei x eine ganze Zahl aus dem Bereich von 100 bis 160 ist, oder eine Variante dieses Antigens, in der höchstens 5 Aminosäuren des HBcAg₁₋ₓ deletiert, insertiert, substituiert oder am C- und/oder N-terminalen Ende angehängt worden sind,
ii) ein Adjuvans beinhaltend ein Saponin, wobei das Adjuvans ein Saponinkomplex ist,
sowie
iii) ein HBsAg (HBsAg = Hepatitis B surface antigen) oder eine Variante dieses Antigens, in der höchstens 5 Aminosäuren des HBsAg deletiert, insertiert, substituiert oder an den Enden angehängt worden sind.

2. Die Zusammensetzung nach Anspruch 1, wobei x eine ganze Zahl aus dem Bereich von 140 bis 149 ist.

3. Die Zusammensetzung nach Anspruch 1 oder 2, wobei x eine ganze Zahl aus dem Bereich von 144 bis 146 ist.

4. Die Zusammensetzung nach einem der vorhergehenden Ansprüche, wobei das Adjuvans als von Wasser oder wässrigen Salzlösungen verschiedene Komponenten
(α1) 0 bis 95 Gew.-% eines Phospholipids,
(α2) 0 bis 95 Gew.-% eines Steroids,
(α3) 5 bis 100 Gew.-% des Saponins oder Saponinderivates, sowie
(α4) 0 bis 20 Gew.-% weitere Zusatzstoffe
beinhaltet, wobei die Gewichtsmengen jeweils auf das Gesamtgewicht des Saponinkomplexes, mit Ausnahme seines Wasseranteils bzw. seines Anteils an wässrigen Salzlösungen, bezogen sind und die Summe der Komponenten (α1) bis (α4) 100 Gew.-% beträgt.

5. Die Zusammensetzung nach einem der vorhergehenden Ansprüche, wobei das relative Mengenverhältnis zwischen den Antigenkomponenten i) und iii) zum Adjuvans ii) in einem Bereich von 1 : 20 bis 20 : 1 liegt.

6. Die Zusammensetzung nach einem der vorhergehenden Ansprüche, wobei die Gesamtkonzentration der Komponenten i), ii) und iii) in der Zusammensetzung in einem Bereich von 0,1 bis 2.000 µg/ml liegt.

7. Die Zusammensetzung nach Anspruch 1, umfassend
i) ein HBcAg₁₋₁₄₅,
ii) einen Saponinkomplex, sowie
iii) ein HBsAg..

8. Die Zusammensetzung nach Anspruch 1, umfassend
i) ein HBcAg_{1-144+I},
ii) einen Saponinkomplex, sowie
iii) HBsAg.

9. Ein Verfahren zur Herstellung einer Zusammensetzung, umfassend die Verfahrensschritte I) - IV):
I) Bereitstellen eines HBcAg₁₋ₓ's, wobei x eine ganze Zahl aus dem Bereich von 100 bis 160 ist, oder einer Variante dieses Antigens, in der höchstens 5 Aminosäuren des HBcAg₁₋ₓ deletiert, insertiert, substituiert oder am C- und/oder N-terminalen Ende angehängt worden sind,
II) Bereitstellen eines Adjuvans beinhaltend ein Saponin, wobei das Adjuvans ein Saponinkomplex ist,
III) Bereitstellen eines HBsAg's oder einer Variante dieses Antigens, in der höchstens 5 Aminosäuren des HBsAg deletiert, insertiert, substituiert oder an den Enden angehängt worden sind, und
IV) In Kontakt bringen des HBcAg₁₋ₓ's, des Adjuvans und des HBsAg's.

10. Das Verfahren nach Anspruch 9, wobei x eine ganze Zahl aus dem Bereich von 140 bis 149 ist.

11. Das Verfahren nach Anspruch 9 oder 10, wobei x eine ganze Zahl aus dem Bereich von 144 bis 146 ist.

12. Das Verfahren nach einem der Ansprüche 9 bis 11, wobei das Adjuvans als von Wasser oder wässrigen Salzlösungen verschiedene Komponenten
(α1) 0 bis 95 Gew.-% eines Phospholipids,
(α2) 0 bis 95 Gew.-% eines Steroids,
(α3) 20 bis 100 Gew.-% des Saponins oder Saponinderivates, sowie
(α4) 0 bis 20 Gew.-% weitere Zusatzstoffe
beinhaltet, wobei die Gewichtsmengen jeweils auf das Gesamtgewicht des Saponinkomplexes, mit Ausnahme seines Wasseranteils bzw. seines Anteils an wässrigen Salzlösungen, bezogen sind und die Summe der Komponenten (α1) bis (α4) 100 Gew.-% beträgt.

13. Eine Zusammensetzung, erhältlich durch ein Verfahren nach einem der Ansprüche 9 bis 12.

14. Eine Arzneimittelformulierung, umfassend die Zusammensetzung nach einem der Ansprüche 1 bis 8 oder 13 sowie geeignete Zusatzstoffe.

15. Die Verwendung der Zusammensetzung nach einem der Ansprüche 1 bis 8 oder 13 zur Herstellung einer Arzneimittelformulierung zur Therapie und/oder zur Prophylaxe von HBV-Infektionen und HBV-vermittelten Erkrankungen.

16. Die Verwendung der Zusammensetzung nach einem der Ansprüche 1 bis 8 oder 13 zur Herstellung einer Arzneimittelformulierung zur Behandlung chronischer HBV-Infektionen.

17. Die Verwendung nach Anspruch 15 oder 16, wobei die HBV-Infektion oder die HBV-vermittelte Erkrankung durch einen HBV-Virus verursacht wurde, dessen Genotyp oder Subtyp sich vom Genotyp oder Subtyp des in der Zusammensetzung enthaltenen HBsAg unterscheidet.

## Revendications

1. Composition comprenant :
i) un HBcAg₁₋ₓ (HBcAg = antigène de core de l'hépatite B), où x représente un nombre entier situé dans la plage de 100 à 160, ou un variant de cet antigène dans lequel au plus 5 acides aminés du HBcAg₁₋ₓ ont été délétés, insérés, substitués ou ajoutés aux extrémités C- et/ou N-terminales,
ii) un adjuvant comprenant une saponine, où l'adjuvant est un complexe de saponines et
iii) un HBsAg (HBsAg = antigène de surface de l'hépatite B) ou un variant de cet antigène, dans lequel au plus 5 acides aminés du HBsAg ont été délétés, insérés, substitués ou ajoutés aux extrémités.

2. Composition selon la revendication 1, où x représente un nombre entier situé dans la plage de 140 à 149.

3. Composition selon la revendication 1 ou la revendication 2, où x représente un nombre entier situé dans la plage de 144 à 146.

4. Composition selon l'une quelconque des revendications précédentes, dans laquelle l'adjuvant comprend en tant que composants qui sont différents de l'eau ou de solutions aqueuses salines
(α1) 0 à 95 % en poids d'un phospholipide,
(α2) 0 à 95 % en poids d'un stéroïde,
(α3) 5 à 100 % en poids de la saponine ou du dérivé de saponine, et
(α4) 0 à 20 % en poids d'autres additifs,
où les quantités en poids sont basées respectivement sur le poids total du complexe de saponines, à l'exception de sa partie eau ou de sa partie solutions aqueuses salines et la somme des composants (α1) à (α4) est de 100 % en poids.

5. Composition selon l'une quelconque des revendications précédentes, dans laquelle le rapport des quantités relatives des composants antigéniques i) et iii) par rapport à l'adjuvant ii) se situe au sein d'une plage de 1/20 à 20/1.

6. Composition selon l'une quelconque des revendications précédentes, dans laquelle la concentration totale des composants i), ii) et iii) dans la composition se situe dans la plage de 0,1 à 2000 µg/ml.

7. Composition selon la revendication 1, comprenant
i) un HBcAg₁₋₁₄₅,
ii) un complexe de saponines, et
iii) un HBsAg.

8. Composition selon la revendication 1, comprenant
i) un HBcAg_{1-144+I},
ii) un complexe de saponines, et
iii) un HBsAg.

9. Procédé de préparation d'une composition, comprenant les étapes de procédé I) à IV) :
I) la fourniture d'un HBcAg₁₋ₓ, où x représente un nombre entier situé dans la plage de 100 à 160, ou d'un variant de cet antigène dans lequel au plus 5 acides aminés du HBcAg₁₋ₓ ont été délétés, insérés, substitués ou ajoutés aux extrémités C- et/ou N-terminales,
II) la fourniture d'un adjuvant comprenant une saponine, où l'adjuvant est un complexe de saponines,
III) la fourniture d'un HBsAg ou d'un variant de cet antigène, dans lequel au plus 5 acides aminés du HBsAg ont été délétés, insérés, substitués ou ajoutés aux extrémités, et
IV) la mise en contact du HBcAg₁₋ₓ, de l'adjuvant et du HBsAg.

10. Procédé selon la revendication 9, dans lequel x représente un nombre entier situé dans la plage de 140 à 149.

11. Procédé selon la revendication 9 ou 10, dans lequel x représente un nombre entier situé dans la plage de 144 à 146.

12. Procédé selon l'une quelconque des revendications 9 à 11, dans lequel l'adjuvant comprend en tant que composants qui sont différents de l'eau ou de solutions aqueuses salines
(α1) 0 à 95 % en poids d'un phospholipide,
(α2) 0 à 95 % en poids d'un stéroïde,
(α3) 20 à 100 % en poids de la saponine ou de dérivé de saponines, et
(α4) 0 à 20 % en poids d'autres additifs,
où les quantités en poids sont basées respectivement sur le poids total du complexe de saponines, à l'exception de sa partie eau ou de sa partie solutions aqueuses salines et la somme des composants (α1) à (α4) est de 100 % en poids.

13. Composition susceptible d'être obtenue par un procédé selon l'une quelconque des revendications 9 à 12.

14. Formulation pharmaceutique, comprenant la composition selon l'une quelconque des revendications 1 à 8 ou 13 et des additifs appropriés.

15. Utilisation de la composition selon l'une quelconque des revendications 1 à 8 ou 13, pour la production d'une formulation pharmaceutique destinée au traitement et/ou à la prophylaxie d'infections par le VHB et de maladies médiées par le VHB.

16. Utilisation de la composition selon l'une quelconque des revendications 1 à 8 ou 13, pour la préparation d'une formulation pharmaceutique destinée au traitement d'infections chroniques par le VHB.

17. Utilisation selon la revendication 15 ou 16, où l'infection par le VHB ou la maladie médiée par le VHB a été provoquée par un virus HB dont le génotype ou le soustype diffère du génotype ou du soustype du HBsAg compris dans la composition.
